# DEMANDE DE BREVET EUROPEEN

(11) **EP 0 842 653 A1**
(43) Date de publication de la demande: **20.05.1998**
(21) Numéro de dépôt: 97402315.2
(22) Date de dépôt: 02.10.1997
(51) Int. Cl.: A61K 7/00

(54) **Utilisation de nanoémulsions à base de lipides amphiphiles non-ioniques fluides pour les fibres kératiniques**

(30) Priorité: 15.11.1996 FR 9613979; 18.03.1997 FR 9703282
(71) Demandeur: L'OREAL, 75008 Paris (FR)
(72) Inventeur: Cauwet-Martin, Danièle, 75011 Paris (FR)
(74) Mandataire: Andral, Christophe André Louis

(57) **Abrégé**

La présente demande concerne l'utilisation pour les fibres kératiniques d'une émulsion huile-dans-eau dont les globules d'huile ont une taille moyenne inférieure à 150 nm, comprenant une phase lipidique amphiphile contenant au moins un lipide amphiphile non-ionique liquide à température ambiante inférieure à 45°C. Cette émulsion peut contenir des quantités importantes d'huile sans apporter les inconvénients généralement connues des huiles tels qu'un toucher gras et chargé.

## Description

La présente invention a trait à l'utilisation pour les fibres kératiniques telles que les cheveux ou les cils d'une émulsion huile-dans-eau dont les globules d'huile ont une taille moyenne inférieure à 150 nm, comprenant une phase lipidique amphiphile contenant au moins un lipide amphiphile non-ionique liquide à température ambiante inférieure à 45°C.

Les émulsions huile-dans-eau sont bien connues dans le domaine des compositions capillaires. Cependant, la présence de concentrations importantes d'huiles végétales, animales ou minérales dans des compositions rend leur formulation difficile. En effet, les compositions sont généralement instables au stockage et les propriétés cosmétiques sont insuffisantes. En particulier, l'application de telles compositions sur les cheveux entraîne un toucher gras et une difficulté de rinçage. De plus, les cheveux séchés sont sans volume et ont un toucher chargé.

On connaît dans l'état de la technique des microémulsions transparentes. Les microémulsions ne sont pas à proprement parler des émulsions ; ce sont des solutions transparentes de micelles, c'est-à-dire que l'huile présente y est solubilisée par la présence conjointe à une forte proportion de tensioactifs et de co-tensioactifs. La taille extrêmement petite des particules, cause de leur transparence, provient de cette «solubilisation». Les inconvénients de ces microémulsions sont justement liés à leur forte proportion en tensioactifs, conduisant à des intolérances et entraînant un toucher désagréable, car non naturel. Ainsi, le document EP-A-334 777 décrit des microémulsions contenant une huile, et un mélange de tensioactif et de co-tensioactif, dans un rapport pondéral huile/mélange de tensioactif et co-tensioactif inférieur à 0,5, c'est à dire avec au moins deux fois plus de tensioactifs que d'huile.

On connaît des nanoparticules comprenant une phase lipidique amphiphile comprenant au moins un phospholipide (lipide amphiphile anionique) et un lipide cationique. Ces nanoparticules peuvent être utilisées dans les soins capillaires. Cependant, les compositions décrites dans ce document sont instables et n'apportent pas de bonnes propriétés cosmétiques, en particulier elles conduisent à un toucher gras et chargé.

On connaît également dans la demande française 2730932 des émulsions huile-dans-eau ayant des globules d'huile dont la taille moyenne est inférieure à 150 nm et comprenant une phase lipidique amphiphile comprenant au moins un lipide amphiphile non-ionique liquide à une température ambiante inférieure à 45°C. Ce document ne décrit ni ne suggère une éventuelle utilisation capillaire.

La demanderesse a découvert, de façon inattendue, que les nanoémulsions décrites dans la demande française 2730932 ne présentent pas les inconvénients des émulsions et des microémulsions dans les compositions utilisées pour le traitement des cheveux. Elles peuvent contenir des quantités importantes d'huile tout en conservant de bonnes propriétés cosmétiques telles que l'absence de toucher gras et chargé.

La présente invention a donc pour objet l'utilisation pour les fibres kératiniques telles que les cheveux ou les cils d'émulsions huile-dans-eau ayant des globules d'huile dont la taille moyenne est inférieure à 150 nm et comprenant une phase lipidique amphiphile comprenant au moins un lipide amphiphile non-ionique liquide à une température ambiante inférieure à 45°C et dont le rapport pondéral de la quantité d'huile sur la quantité de la phase lipidique amphiphile étant compris entre 2 et 10.

Par utilisation pour les fibres kératiniques telles que les cheveux, les cils ou les sourcils, on entend selon la présente invention l'application de l'émulsion sur les cheveux ou les cils ou les sourcils pour leur lavage et /ou leur traitement et/ou leur maintien.

Les lipides amphiphiles non-ioniques de l'invention sont préférentiellement choisis parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée. On peut également utiliser les mélanges de des composés ci-dessus.

Les tensioactifs siliconés utilisables selon l'invention sont des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5364633 et US-A-5411744.

De préférence, le tensioactif siliconé utilisé selon la présente invention est un composé de formule (I) ; 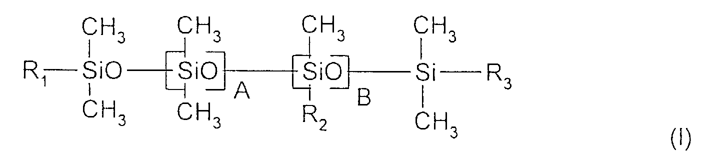 dans laquelle :
R₁, R₂, R₃, indépendamment les uns des autres, représentent un radical alkyle en C₁-C₆ ou un radical -(CH₂)ₓ -(OCH₂CH₂)_{y} -(OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 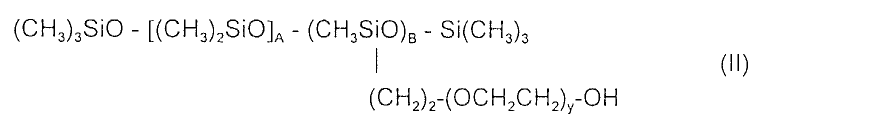 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (I), les composés de formule (III) :

HO - (OCH₂CH₂)_{y}-(CH₂)₃ - [(CH₃)₂SiO]_{A'} - (CH₂)₃ - (OCH₂CH₂)_{y} - OH (III)

dans laquelle A' et y sont des nombres entiers allant de 10 à 20.

On peut utiliser comme composés de l'invention ceux commercialisés par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.

Le composé Q4-3667 est un composé de formule (III) où A est 15 et y est 13.

Parmi les lipides amphiphiles non ioniques, on peut plus particulièrement citer, à titre d'exemple :
- l'isostéarate de polyéthylèneglycol de poids moléculaire 400,
- l'isostéarate de diglycéryle,
- le laurate de polyglycérol comportant 10 unités de glycérol ,
- l'oléate de sorbitane,
- l'isostéarate de sorbitane,
- le cocoate d'α-butylglucoside ou le caprate d'α-butylglucoside.

Le rapport pondéral de la quantité d'huile contenue dans l'émulsion conforme à l'invention sur la quantité de la phase lipidique amphiphile varie, de préférence, de 2 à 10 et plus préférentiellement de 3 à 6.

Une forme particulière d'émulsion conforme à l'invention est caractérisée par le fait que la phase lipidique amphiphile comprend en plus un ou plusieurs lipides amphiphiles ioniques.

Les lipides amphiphiles ioniques, utilisés dans les nanoémulsions de l'invention, sont choisis, de préférence, dans le groupe formé par les lipides anioniques, les lipides amphotères et leurs mélanges.

Les lipides amphiphiles anioniques sont plus particulièrement choisis dans le groupe formé par:
- les sels alcalins du dicétyl- et du dimyristylphosphate ;
- les sels alcalins du cholestérol sulfate ;
- les sels alcalins du cholestérol phosphate ;
- les lipoaminoacides tels que les acylglutamates mono et disodiques ;
- les sels de sodium de l'acide phosphatidique ;
- les phospholipides ;
- les dérivés alkylsulfoniques tels que ceux de formule : 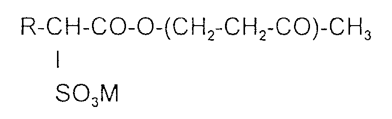
dans laquelle R représente des radicaux alkyle en C₁₆-C₂₂, en particulier les radicaux C₁₆H₃₃ et C₁₈H_{37,} pris en mélange ou séparément et M est un métal alcalin tel que le sodium.

Les lipides ioniques amphiphiles sont présents dans les nanoémulsions de l'invention, de préférence, dans des concentrations allant de 1 à 60% en poids et plus particulièrement de 10 à 50 % en poids par rapport au poids total de la phase lipidique amphiphile.

Le rapport pondéral de la quantité d'huile contenue dans l'émulsion conforme à l'invention sur la quantité de la phase lipidique amphiphile est compris, de préférence entre 3 à 6.

Les nanoémulsions conformes à l'invention comportent une quantité d'huile allant de préférence, de 5 à 40% en poids par rapport au poids total de l'émulsion.

Les huiles pouvant être utilisées dans les émulsions de l'invention sont choisies préférentiellement dans le groupe formé par :
- les huiles animales ou végétales, notamment celles formées par des esters d'acide gras et de polyols, en particulier les triglycérides liquides, par exemple les huiles de tournesol, d'avocat, de maïs, de soja, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée contenant de 3 à 30 atomes de carbone, par exemple, l'huile de Purcellin ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- des hydrocarbures, tels que l'hexadécane et l'huile de paraffine ;
- des carbures halogénés, notamment des fluorocarbures tels que des fluoramines par exemple la perfluorotributylamine, des hydrocarbures fluorés, par exemple le perfluorodécahydronaphtalène, des fluoroesters et des fluoroéthers ;
- des esters d'acide minéral et d'un alcool ;
- des éthers et des polyéthers ;
- des silicones en mélange avec au moins l'une des huiles définies ci-dessus, par exemple le décaméthylcyclopentasiloxane ou le dodécaméthylcyclohexasiloxane.

Les émulsions conformes à la présente invention peuvent contenir des additifs pour améliorer la transparence de la formulation, dans des concentrations comprises entre 1 et 30% en poids par rapport au poids total de l'émulsion et de préférence entre 5 et 20% en poids.

Ces additifs sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.

En outre, l'utilisation des alcools tels que définis ci-dessus, à des concentrations supérieures ou égales à 5% en poids et de préférence supérieure à 15%, permet d'obtenir des émulsions sans conservateur.

Les émulsions de l'invention peuvent contenir des actifs hydrosolubles ou liposolubles, ayant une activité cosmétique ou dermopharmaceutique. Les actifs liposolubles sont dans les globules huileux de l'émulsion, tandis que les actifs hydrosolubles sont dans la phase aqueuse de l'émulsion. On peut citer, à titre d'exemples d'actif, les vitamines telles que la vitamine E et ses dérivés, les provitamines telles que le panthénol, les humectants, les filtres solaires, des agents tensioactifs, des agents conservateurs, des séquestrants, des adoucissants, des parfums, des colorants, des agents modificateurs de viscosité, des agents modificateurs de mousse, des stabilisateurs de mousse, des agents nacrants, des agents hydratants, des agents antipelliculaires, des agents antiséborrhéiques, des protéines, des silicones, des céramides, des pseudocéramides, des acides gras à chaînes linéaires ou ramifiées en C₁₆-C₄₀ tels que l'acide méthyl-18 eicosanoique, des épaississants, des plastifiants, des hydroxyacides, des électrolytes et des parfums.

Parmi les épaississants utilisables, on peut citer les dérivés de cellulose tels que l'hydroxyméthylpropylcellulose, les alcools gras tels que les alcools stéarylique, cétylique, béhénique, les dérivés d'algues tels que le satiagum, des gommes naturelles telles que l'adragante et des polymères synthétiques tels que les mélanges d'acides polycarboxyvinyliques commercialisés sous la dénomination CARBOPOL par la société GOODRlCH et le mélange de copolymères acrylate de Na/acrylamide commercialisé sous la dénomination HOSTACERIN PN 73 par la société HOECHST.

La taille moyenne des globules d'huile est généralement comprise entre 30 et 150 nm, de préférence entre 40 et 100 nm et encore plus particulièrement entre 50 et 80 nm.

Les émulsions selon l'invention sont incolores et éventuellement légèrement bleutées et présentent une transparence déterminée par le coefficient de transmittance mesuré à une longueur d'onde de 600 nm, allant, préférentiellement, de 10 à 90% et plus particulièrement de 20 à 80%.

Les nanoémulsions de l'invention peuvent être obtenues par un procédé, caractérisé par le fait qu'on mélange la phase aqueuse et la phase huileuse , sous agitation vive, à une température ambiante inférieure à 45°C puis qu'on effectue une homogénéisation haute pression à une pression supérieure à 10⁸ Pa et de préférence allant de 12.10⁷ à 18.10⁷ Pa. Un tel procédé permet de réaliser, à température ambiante, des nanoémulsions compatibles avec des composés actifs thermosensibles.

Les compositions conformes à l'invention peuvent être utilisées pour le lavage, le maintien et le traitement des fibres kératiniques telles que les cheveux et les cils et plus particulièrement les cheveux.

Les compositions de l'invention peuvent plus particulièrement se présenter sous forme de shampooings, d'après-shampooings à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage.

Les compositions peuvent être également des lotions de mise en plis, des lotions pour le brushing, des compositions de fixation (laques) et de coiffage telles que par exemple des gels, ou des mousses. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Les compositions utilisées selon l'invention peuvent également être des compositions de maquillage ou de soin des cils ou des sourcils telles que les mascaras.

Un autre objet de l'invention consiste en une composition pour les fibres kératiniques et en particulier une composition capillaire, caractérisée par le fait qu'elle est constituée ou qu'elle comprend une émulsion telle que définie précédemment.

Par composition capillaire, on entend selon la présente invention une composition destinée à être appliquée sur les cheveux pour leur lavage et/ou leur traitement et/ou leur maintien.

Enfin, l'invention porte également sur un procédé non-thérapeutique de traitement des fibres kératiniques telles que les cheveux, caractérisé par le fait qu'on applique sur les fibres kératiniques une émulsion telle que définie ci-dessus ou une composition comprenant une telle émulsion.

Les exemples qui suivent, permettront de mieux comprendre l'invention, sans toutefois présenter un caractère limitatif.

### EXEMPLES

Pour l'exemple 1, le mode opératoire suivant est mis en oeuvre :
- dans une première phase A, on homogénéise les lipides amphiphiles avec les huiles et les actifs et adjuvants lipophiles à une température de 45°C ;
- dans une seconde phase B, on dissout les actifs et adjuvants hydrophiles à une température de 20 à 30°C ;
- puis, on mélange les phases A et B à l'aide d'un homogénéisateur à turbine puis on homogénéise à l'aide d'un homogénéisateur à haute pression du type Soavi-Niro à une pression de 1500 bars, en 7 passages en maintenant la température du produit en dessous de 35°C.

### EXEMPLE 1 : Nanoémulsion d'huile d'avocat

### Première phase :

- Isostéarate de PEG-400, vendu par la société UNICHEMA 4,5 %
- Sel disodique de l'acide N-stéaroyl L-glutamique commercialisé sous la dénomination Acylglutamate HS21 par la Société AJINOMOTO (lipide amphiphile ionique) 0,5 %
- Huile d'avocat 20 %
- Ethanol absolu non dénaturé 15 %

### Deuxième phase :

- Eau déminéralisée 54,7 %
- Glycérine 5 %

On obtient une émulsion dont la taille des globules d'huile est d'environ 63 nm

### EXEMPLE 2 : Emulsion comparative

On a préparé une émulsion par un mode opératoire classique avec les mêmes ingrédients que dans la nanoémulsion de l'exemple 1.

On a appliqué 5 g d'émulsion ou de nanoémulsion sur des mèches de 2,5 g. On a laissé poser 10 mn puis on a effectué un rinçage sous l'eau du robinet. On a fait sécher les mèches pendant 30 mn sous un casque séchoir.

On a ensuite demandé à un panel de 10 experts de juger le toucher et l'aspect des cheveux.

Les 10 experts à l'unanimité ont préféré la mèche traitée avec la nanoémulsion. Le toucher est non gras et l'aspect est naturel.

Cet effet est d'autant plus surprenant que la quantité d'huile déposée sur les cheveux est plus importante lorsqu'on utilise la nanoémulsion.

## Revendications

1. Utilisation pour les fibres kératiniques d'une émulsion huile-dans-eau dont les globules d'huile ont une taille moyenne inférieure à 150 nm et comprenant une phase lipidique amphiphile, comprenant au moins un lipide amphiphile non-ionique liquide à une température ambiante inférieure à 45°C et que le rapport pondéral de la quantité d'huile sur la quantité de phase lipidique amphiphile est compris entre 2 et 10.

2. Utilisation selon la revendication 1, caractérisée par le fait que le lipide amphiphile non-ionique est choisi parmi les tensioactifs siliconés et les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée, et leurs mélanges.

3. Utilisation selon la revendication 2, caractérisée en ce que le tensioactif siliconé est un composé de formule (I) ; 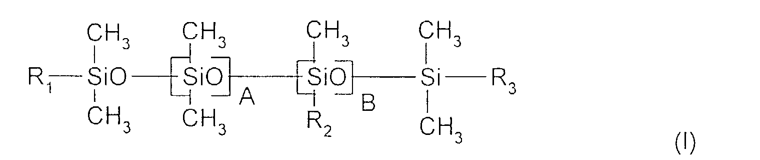 dans laquelle
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

4. Utilisation selon la revendication 2, caractérisée en ce que le tensioactif siliconé est un composé de formule (II) : 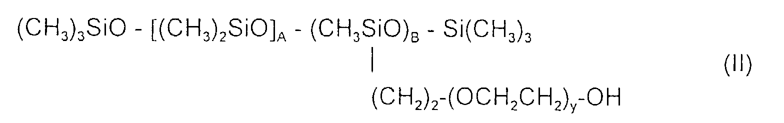 dans laquelle A est un nombre entier allant de 20 à 105, B est un nombre entier allant de 2 à 10 et y est un nombre entier allant de 10 à 20.

5. Utilisation selon la revendication 2, caractérisée en ce que le tensioactif siliconé est un composé de formule (III) :
HO - (OCH₂CH₂)_{y}-(CH₂)₃ - [(CH₃)₂SiO]_{A'} - (CH₂)₃ - (OCH₂CH₂)_{y} - OH (III)
dans laquelle A' et y sont des nombres entiers allant de 10 à 20.

6. Utilisation selon l'une quelconque des revendications 1 à 5 , caractérisée par le fait que le rapport pondéral de la quantité d'huile sur la quantité de phase lipidique amphiphile est compris entre 3 et 6.

7. Utilisation selon l'une quelconque des revendications 1 à 6, caractérisée par le fait que la phase lipidique amphiphile contient en plus au moins un lipide amphiphile ionique.

8. Utilisation selon la revendication précédente, caractérisée en ce que le lipide amphiphile ionique est choisi dans le groupe formé par les lipides anioniques, les lipides amphotères et leurs mélanges.

9. Utilisation selon l'une des revendication 7 à 8, caractérisée par le fait que le lipide amphiphile ionique est présent dans des concentrations allant de 1 à 60 % en poids par rapport au poids total de la phase lipidique amphiphile.

10. Utilisation selon l'une quelconque des revendications 1 à 9, caractérisée par le fait qu'elle comprend une proportion d'huile allant de 5 à 40% en poids par rapport au poids total de l'émulsion.

11. Utilisation selon l'une quelconque des revendications 1 à 10, caractérisée par le fait que l'huile est choisie dans le groupe formé par :
- les huiles animales ou végétales, notamment formées par des esters d'acides gras et de polyols ou bien les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée contenant de 3 à 30 atomes de carbone ;
- les huiles essentielles naturelles ou synthétiques ;
- les hydrocarbures ;
- les carbures halogénés ;
- les esters d'acide minéral et d'alcool ;
- les éthers et polyéthers ;
- les silicones en mélange avec au moins l'une des huiles définies ci-dessus.

12. Utilisation selon l'une quelconque des revendications 1 à 11, caractérisée par le fait qu'elle contient un actif cosmétique ou dermopharmaceutique, hydrosoluble ou liposoluble.

13. Utilisation selon l'une quelconque des revendications 1 à 12, caractérisée par le fait que les globules d'huile ont une taille moyenne allant de 30 à 150 nm.

14. Composition pour les fibres kératiniques, caractérisée par le fait qu'elle est constituée ou qu'elle comprend une émulsion selon l'une quelconque des revendications 1 à 11.

15. Procédé de traitement non-thérapeutique des fibres kératiniques, caractérisé par le fait qu'on applique sur les fibres kératiniques une émulsion selon l'une quelconque des revendications 1 à 13.
